(19) Europäisches Patentamt
European Patent Office
Office européen des brevets

(11) **EP 4 213 093 B1**

(12) **EUROPEAN PATENT SPECIFICATION**

(45) Date of publication and mention
of the grant of the patent:
**04.03.2026 Bulletin 2026/10**

(21) Application number: **23151807.7**

(22) Date of filing: **16.01.2023**

(51) International Patent Classification (IPC):
*G06T 7/00* *(2017.01)*

(52) Cooperative Patent Classification (CPC):
**G06T 7/0012;** G06T 2207/10132;
G06T 2207/20081; G06T 2207/30096

(54) **MEDICAL IMAGE PROCESSING DEVICE, ULTRASONIC DIAGNOSTIC APPARATUS, AND PROGRAM**

VORRICHTUNG ZUR VERARBEITUNG MEDIZINISCHER BILDER, ULTRASCHALLDIAGNOSEVORRICHTUNG UND PROGRAMM

DISPOSITIF DE TRAITEMENT D'IMAGE MÉDICALE, APPAREIL DE DIAGNOSTIC ULTRASONORE ET PROGRAMME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **18.01.2022 JP 2022005759**

(43) Date of publication of application:
**19.07.2023 Bulletin 2023/29**

(73) Proprietor: **Canon Medical Systems Corporation
Tochigi (JP)**

(72) Inventors:
• **YOSHIARA, Hiroki
Otawara-shi, 324-0036 (JP)**
• **WATANABE, Masaki
Otawara-shi, 324-0036 (JP)**

(74) Representative: **Marks & Clerk LLP
15 Fetter Lane
London EC4A 1BW (GB)**

(56) References cited:
• **LIANG XIAODAN ET AL: "Recognizing Focal
Liver Lesions in CEUS With Dynamically Trained
Latent Structured Models", IEEE
TRANSACTIONS ON MEDICAL IMAGING, IEEE,
USA, vol. 35, no. 3, 1 March 2016 (2016-03-01),
pages 713 - 727, XP011608604, ISSN: 0278-0062,
[retrieved on 20160301], DOI: 10.1109/
TMI.2015.2492618**
• **KONDO SATOSHI ET AL: "Computer-Aided
Diagnosis of Focal Liver Lesions Using Contrast-
Enhanced Ultrasonography With Perflubutane
Microbubbles", IEEE TRANSACTIONS ON
MEDICAL IMAGING, IEEE, USA, vol. 36, no. 7, 1
July 2017 (2017-07-01), pages 1427 - 1437,
XP011654766, ISSN: 0278-0062, [retrieved on
20170628], DOI: 10.1109/TMI.2017.2659734**

EP 4 213 093 B1

**Description**

FIELD

Embodiments disclosed in the present specification and drawings relate to a medical image processing device, an ultrasonic diagnostic apparatus, and a program.

BACKGROUND

There is a diagnostic technology for capturing an image (video) of a subject and detecting lesions on the basis of the obtained image. In this technology, for example, a contrast medium is administered to a subject, and lesions are detected on the basis of how the administered contrast medium stains, and the like. For example, a site that has been washed out after being stained with the contrast medium and has become defective is detected as a lesion.

Although an operator selects an appropriate image from, for example, a plurality of frames in order to view how the contrast medium stains, the number of images to be selected is enormous and thus effort is required to select an appropriate image. If an appropriate image cannot be selected, it is difficult to appropriately detect lesions.

Liang X, Lin L, Cao Q, Huang R, Wang Y. Recognizing focal liver lesions in CEUS with dynamically trained latent structured models. IEEE Transactions on Medical Imaging. 2015 Oct 26;35(3):713-27 generally relates to automatically classifying Focal Liver Lesions (FLLs) into three specific benign or malignant types in Contrast-Enhanced Ultrasound (CEUS) videos, and aims at providing a computational framework to assist clinicians in FLL diagnosis. The main challenge for this task is that FLLs in CEUS videos often show diverse enhancement patterns at different temporal phases. To handle these diverse patterns, a model is proposed which detects a number of discriminative Regions of Interest (ROIs) for the FLL and recognize the FLL based on these ROIs. The model incorporates an ensemble of local classifiers in the attempt to identify different enhancement patterns of ROIs, and in particular, the model is made reconfigurable by introducing switch variables to adaptively select appropriate classifiers during inference.

Kondo S, Takagi K, Nishida M, Iwai T, Kudo Y, Ogawa K, Kamiyama T, Shibuya H, Kahata K, Shimizu C. Computer-aided diagnosis of focal liver lesions using contrast-enhanced ultrasonography with perflubutane microbubbles. IEEE transactions on medical imaging. 2017 Jan 26;36(7):1427-37. generally relates to an automatic classification method based on machine learning in contrast-enhanced ultrasonography (CEUS) of focal liver lesions using the contrast agent Sonazoid. This method yields spatial and temporal features in the arterial phase, portal phase, and post-vascular phase, as well as max-hold images. The lesions are classified as benign or malignant and again as benign, hepatocellular carcinoma (HCC), or metastatic liver tumor using support vector machines (SVM) with a combination of selected optimal features.

SUMMARY

An object of embodiments disclosed in the present specification and drawings is to reduce the time required to select an appropriate frame and appropriately detect a lesion. However, the object to be achieved by the embodiments disclosed in the present specification and drawings is not limited to the above objects. An object corresponding to each effect of each configuration shown in the embodiments described later can be positioned as another object.

(1) In a first aspect, a medical image processing device is provided according to claim 1.
(14) In a second aspect, a computer-readable storage medium is provided according to claim 14.

BRIEF DESCRIPTION OF THE DRAWINGS

FIG. 1 is a block diagram showing an example of a configuration of an ultrasonic diagnostic apparatus 1 of a first embodiment.
FIG. 2 is a diagram showing an example of an image displayed on a display 130.
FIG. 3 is a diagram showing an example of phase changes after administration of a contrast medium.
FIG. 4 is a diagram schematically showing a contrast-enhanced image and a tissue image.
FIG. 5 is a diagram showing an example of a TIC.
FIG. 6 is a flowchart showing an example of processing of a medical image processing device 100.
FIG. 7 is a flowchart showing an example of processing of the medical image processing device 100.
FIG. 8 is a flowchart showing an example of processing of updating a first trained model 184.
FIG. 9 is a diagram schematically showing an internal image in an artery dominant phase and an internal image in a portal vein dominant phase of a second embodiment.

FIG. 10 is a diagram schematically showing a tissue image before administration of a contrast medium and internal images in an artery dominant phase and a portal vein dominant phase of a third embodiment.

FIG. 11 is a block diagram showing an example of a configuration of a medical information processing device 300 of a fourth embodiment.

DETAILED DESCRIPTION

Hereinafter, a medical image processing device, an ultrasonic diagnostic apparatus, a medical information processing apparatus, and a program according to embodiments will be described with reference to the drawings.

(First embodiment)

A medical image processing device of a first embodiment is provided, for example, in an ultrasonic diagnostic apparatus. The ultrasonic diagnostic apparatus is a medical apparatus that captures a medical image of a subject. The medical image processing device is included, for example, in an ultrasonic diagnostic apparatus, an X-ray computed tomography (CT) apparatus, a positron emission tomography (PET)-CT apparatus, a magnetic resonance imaging (MRI) apparatus, and the like.

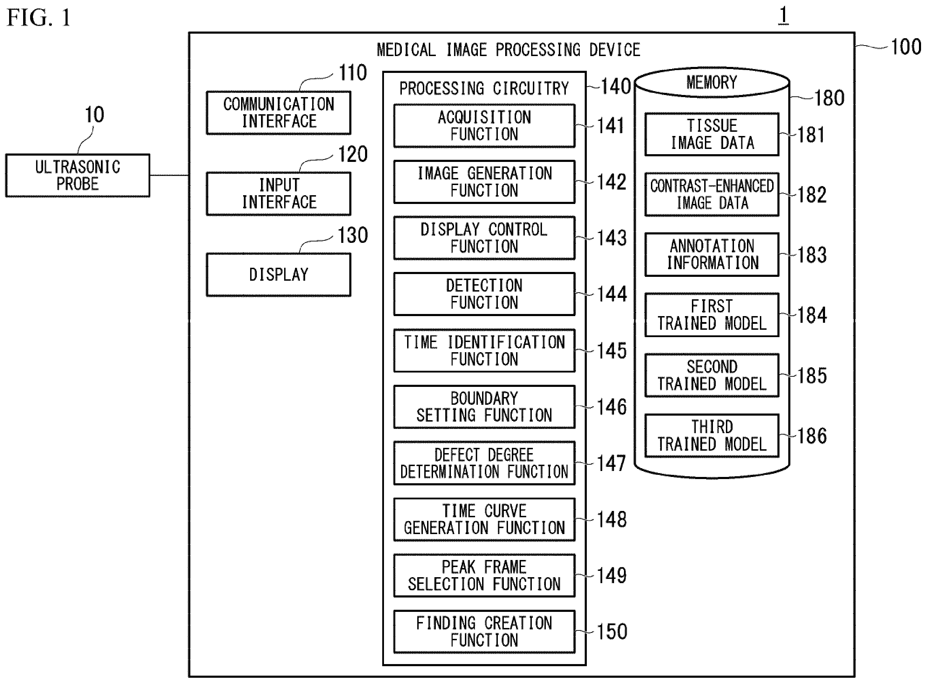

FIG. 1 is a block diagram showing an example of a configuration of the ultrasonic diagnostic apparatus 1 of the first embodiment. The ultrasonic diagnostic apparatus 1 includes, for example, an ultrasonic probe 10 and a medical image processing device 100. The ultrasonic diagnostic apparatus 1 is installed, for example, in a medical institution such as a hospital. The ultrasonic diagnostic apparatus 1 is operated by, for example, an operator such as an engineer or a doctor, and captures and stores medical images of the inside the body of a subject that is a patient. The ultrasonic diagnostic apparatus 1 creates findings with respect to the subject on the basis of the stored medical images.

In an examination using the ultrasonic diagnostic apparatus 1, for example, a contrast medium is administered to a subject, and the presence or absence of a defect is detected according to how the administered contrast medium stains and a washout state. If a defect is detected, findings based on the size of the defect, the presence or absence of a stain in an arterial phase, the presence or absence of washout, a timing and extent of washout, and the like are created. Washout refers to, for example, a decrease in concentration of a contrast medium seen in a contrast-enhanced image. Washout progresses as a stain in a contrast-enhanced image disappears.

The ultrasonic probe 10 is, for example, operated by an operator and pressed against a part of the subject (an examination or diagnosis target site). For example, the ultrasonic probe 10 transmits (radiates) ultrasonic waves to the subject at regular time intervals in order to obtain an image of the inside of the subject. The ultrasonic probe 10 receives echoes (reflected waves) of transmitted ultrasonic waves.

The ultrasonic probe 10 generates data of received echo signals (hereinafter referred to as echo data). The ultrasonic probe 10 outputs the generated echo data to the medical image processing device 100. The ultrasonic probe 10 receives ultrasonic echoes at regular time intervals and generates echo data. Therefore, echo data at regular intervals is output to the medical image processing device 100.

The medical image processing device 100 includes, for example, a communication interface 110, an input interface 120, a display 130, processing circuitry 140, and a memory 180. The communication interface 110 communicates with external devices via a communication network NW. The communication interface 110 includes, for example, a communication interface such as a network interface card (NIC).

The input interface 120 receives various input operations from an operator of the ultrasonic diagnostic apparatus 1, converts the received input operations into electrical signals, and outputs the electrical signals to the processing circuitry 140. For example, the input interface 120 includes a mouse, a keyboard, a trackball, a switch, a button, a joystick, a touch panel, and the like. The input interface 120 may be, for example, a user interface that receives voice input, such as a microphone. When the input interface 120 is a touch panel, the input interface 120 may also have the display function of the display 130.

The input interface in the present specification is not limited to those having physical operation parts such as a mouse and a keyboard. For example, examples of the input interface include an electrical signal processing circuit that receives an electrical signal corresponding to an input operation from external input equipment provided separately from the device and outputs the electrical signal to a control circuit.

The display 130 displays various types of information. For example, the display 130 displays images generated by the processing circuitry 140, a graphical user interface (GUI) for receiving various input operations from the operator, and the like. For example, the display 130 is a liquid crystal display (LCD), a cathode ray tube (CRT) display, an organic electroluminescence (EL) display, or the like.

The processing circuitry 140 includes, for example, an acquisition function 141, an image generation function 142, a display control function 143, a detection function 144, a time identification function 145, a boundary setting function 146, a defect degree determination function 147, a time curve generation function 148, a peak frame selection function 149, and a finding creation function 150. The processing circuitry 140 realizes these functions by, for example, a hardware processor (computer) executing a program stored in the memory 180 (storage circuit).

The hardware processor refers to, for example, a circuitry such as a central processing unit (CPU), a graphics processing unit (GPU), an application specific integrated circuit (ASIC), a programmable logic device (for example, a simple programmable logic device (SPLD), a complex programmable logic device (CPLD), or a field programmable gate array (FPGA)) or the like. Instead of storing the program in the memory 180, the program may be directly embedded in the circuitry of the hardware processor. In this case, the hardware processor realizes the functions by reading and executing the program embedded in the circuitry. The aforementioned program may be stored in the memory 180 in advance, or may be stored in a non-transitory storage medium such as a DVD or CD-ROM and installed in the memory 180 from the non-transitory storage medium when the non-transitory storage medium is set in a drive device (not shown) of the ultrasonic diagnostic apparatus 1. The hardware processor is not limited to being configured as a single circuit and may be configured as one hardware processor by combining a plurality of independent circuits to realize each function. Further, a plurality of components may be integrated into one hardware processor to realize each function.

The memory 180 is realized by, for example, a semiconductor memory element such as a random access memory (RAM) and a flash memory, a hard disk, or an optical disc. These non-transitory storage media may be realized by other storage devices such as a network attached storage (NAS) and an external storage server device connected via the communication network NW. Further, the memory 180 may also include non-transitory storage media such as a read only memory (ROM) and a register. The memory 180 stores tissue image data 181, contrast-enhanced image data 182, annotation information 183, a first trained model 184, a second trained model 185, and a third trained model 186.

The acquisition function 141 acquires imaging data output by echo data output from the ultrasonic probe 10. Time information indicating the time when the ultrasonic probe 10 has received an echo from which the echo data is generated (the time when the imaging data has been captured) is added to the echo data and the imaging data. The acquisition function 141 acquires the time information along with the echo data.

The acquisition function 141 receives input operations performed by the operator on the input interface 120. For example, when a contrast medium is administered to the subject after the ultrasonic diagnostic apparatus 1 starts an examination of the subject, the operator can input administration information. The input interface 120 outputs an electrical signal corresponding to the administration information to the acquisition function 141 when input of the administration information is received. The acquisition function 141 is an example of an acquirer.

The image generation function 142 generates internal images showing the internal state of the subject on the basis of the echo data acquired by the acquisition function 141. The image generation function 142 generates and acquires, as internal images, an image based on echo data of tissue images (hereinafter referred to as a tissue image) and an image based on echo data of the contrast medium (hereinafter referred to as a contrast-enhanced image). The image generation function 142 distinguishes the echo data of the tissue images from the echo data of the contrast medium, for example, on the basis of an imaging technique or an imaging band at the time of imaging the echo data. Time information added to the echo data of the tissue images and the echo data of the contrast medium is added to the internal images. The image generation function 142 is an example of an acquirer.

The image generation function 142 generates an internal video (captured video and contrast-enhanced video) in which, for example, a plurality of internal images (tissue images and contrast-enhanced images) are arranged as frames in chronological order. The image generation function 142 stores the generated tissue images and contrast-enhanced images in the memory 180 as tissue image data 181 and contrast-enhanced image data 182. The tissue image data 181 includes tissue images and tissue video, and the contrast-enhanced image data 182 includes contrast-enhanced images and contrast-enhanced video.

The display control function 143 causes the display 130 to display the internal images after a series of internal images in an examination is generated by the image generation function 142 and the tissue image data 181 and the contrast-enhanced image data 182 are stored in the memory 180. The image generation function 142 generates an internal video on the basis of echo data at regular time intervals. Therefore, an internal video generated by the image generation function 142 is, so to speak, a continuous video.

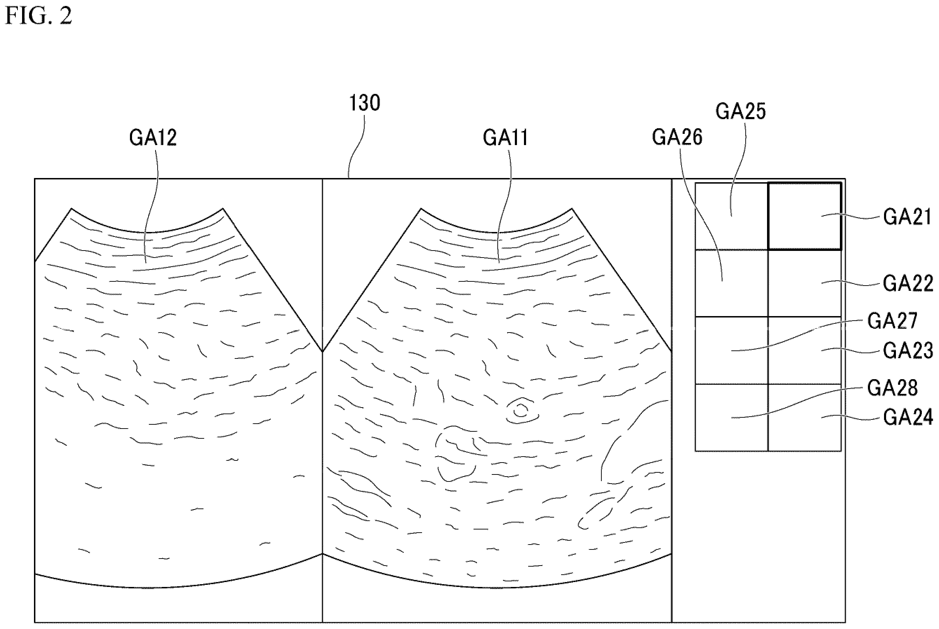

FIG. 2 is a diagram showing an example of an image displayed on display 130. A tissue image GA11 and a contrast-enhanced image GA12 are displayed side by side in the center of the display 130. A first thumbnail image GA21 to an eighth thumbnail image GA28 are displayed in the upper right portion of the display 130.

The first thumbnail image GA21 to the eighth thumbnail image GA28 are samples of internal images for each time period arranged in chronological order after administration of a contrast medium to a subject. Transition of phases after administration of the contrast medium to the subject occurs in the order of an artery dominant phase, a portal vein dominant phase, and a post-vascular phase. For example, the image generation function 142 classifies tissue images and contrast-enhanced images after administration of the contrast medium into the artery dominant phase, the portal vein dominant phase, and the post-vascular phase, generates the images as moving images of each time period, and stores the moving images in the memory 180. An interval between the artery dominant phase and the portal vein dominant phase is, for example, a first intermediate phase, and an interval between the portal vein dominant phase and the post-vascular phase is, for example, a second intermediate phase.

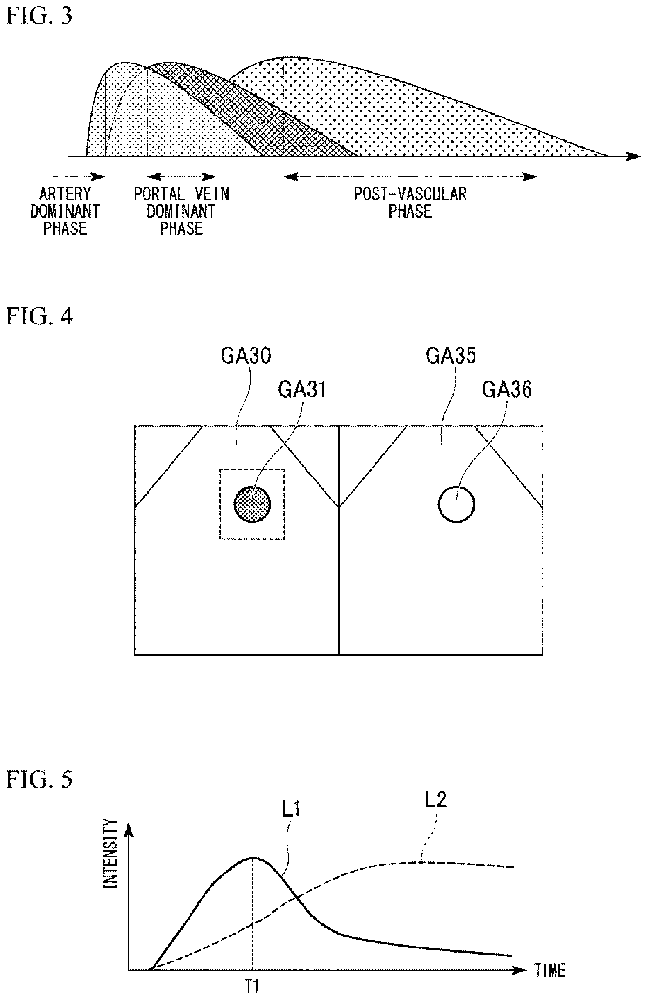

FIG. 3 is a diagram showing an example of phase changes after administration of a contrast medium. An artery dominant phase, a portal vein dominant phase, and a post-vascular phase are defined on the basis of annotation information 183, for example. The annotation information 183 may be information set for an examination process, and the artery dominant phase, portal vein dominant phase, and post-vascular phase may be determined on the basis of time information measured by a timer.

For example, an interval in which time information after administration of the contrast medium (information indicating a time elapsed from administration of the contrast medium) is 10 to 30 seconds is defined as the artery dominant phase, an interval corresponding to 60 to 90 seconds is defined as the portal vein dominant phase, and a time for which 5 minutes or more has elapsed is defined as the post-vascular phase. In the embodiment, the artery dominant phase, the portal vein dominant phase, and the post-vascular phase use time information measured by a timer.

Among contrast-enhanced images, the artery dominant phase is, for example, a video captured during a time period corresponding to the first thumbnail image GA21. The portal vein dominant phase is, for example, a video captured during a time period corresponding to the sixth thumbnail image GA26. The post-vascular phase is, for example, a video captured during a time period corresponding to the eighth thumbnail image GA28. As the first thumbnail image GA21 to the eighth thumbnail image GA28, for example, contrast-enhanced images captured at the beginning of each time period are used. Other images may be used as thumbnail images.

When the operator performs an input operation on any one of the first thumbnail image GA21 to the eighth thumbnail image GA28, an internal image (internal video) of a time period corresponding to the thumbnail image on which the input operation has been performed is displayed in the center of the display 130. For example, when the operator performs an input operation on the first thumbnail image GA21, the tissue image GA11 and contrast-enhanced image GA12 of the artery dominant phase are displayed in the center of the display 130. An internal image displayed in response to an input operation for a thumbnail is, for example, a moving image.

When a tissue image is displayed on the display 130, the operator sets a detection region-of-interest (ROI) at a position where a lesion is suspected in the tissue image. The operator uses the input interface 120 to perform an operation input for setting the detection ROI. The input interface 120 outputs an electrical signal according to the input operation of the operator to the processing circuitry 140. The acquisition function 141 of the processing circuitry 140 acquires the set position of the detection ROI according to the electrical signal output by the input interface 120.

The detection function 144 detects the presence or absence of a defective part on the basis of contrast-enhanced images after the portal vein dominant phase. In detecting a defective part, the detection function 144 utilizes the first trained model 184 stored in the memory. The first trained model 184 is a trained model generated by using, as training data, a plurality of contrast-enhanced images in which defective parts are detected and a plurality of contrast-enhanced images in which defective parts are not detected in contrast-enhanced images after the portal vein dominant phase. The first trained model 184 may be a trained model generated by using, as training data, a plurality of contrast-enhanced images in which defective parts are detected.

The detection function 144 detects washout of the contrast medium on the basis of output results obtained by inputting the contrast-enhanced images after the portal vein dominant phase into the first trained model 184. The detection function 144 detects the presence or absence of a defective part on the basis of the washout state of the contrast medium. The detection function 144 is an example of a detector.

As a result of detecting the presence or absence of a defective part using the contrast-enhanced images after the portal vein dominant phase as input data, if a defective part cannot be detected, the detection function 144 detects the presence or absence of a lesioned part on the basis of output results obtained by inputting tissue images of the artery dominant phase to the second trained model 185 as input data. The second trained model 185 is a trained model generated by using, as training data, a plurality of tissue images in which lesioned parts are detected and a plurality of tissue images in which no lesioned parts are detected in the artery dominant phase. The second trained model 185 may be a trained model generated by using, as training data, a plurality of tissue images in which lesioned parts are detected. Instead of or in addition to tissue images in the artery dominant phase, tissue images after the portal vein dominant phase may be used.

When the detection function 144 has detected a lesioned part on the basis of output results obtained by inputting the tissue images of the artery dominant phase to the second trained model 185 as input data, it is necessary to identify the position of the lesioned part in contrast-enhanced images. Therefore, the detection function 144 selects a frame with a highest likelihood from among the frames of the contrast-enhanced images. The detection function 144 identifies the lesioned part in the contrast-enhanced images on the basis of the position of the lesioned part in the tissue image of the selected frame.

When a lesioned part cannot be detected as a result of detecting the presence or absence of a lesioned part using tissue images of the artery dominant phase as input data, the detection function 144 inputs contrast-enhanced images of the artery dominant phase to the third trained model 186 as input data and detects the present or absence of a lesioned part on the basis of output data output from the third trained model 186. The third trained model 186 is a trained model generated by using, as training data, a plurality of contrast-enhanced images in which lesioned parts are detected and a plurality of contrast-enhanced images in which no lesioned parts are detected in the artery dominant phase. The third trained model 186 may be a trained model generated by using, as training data, a plurality of contrast-enhanced images in which lesioned parts are detected.

When the detection function 144 detects a plurality of defective parts, the display control function 143 causes the display 130 to display a contrast-enhanced image including the plurality of defective parts. When the input interface 120 receives an input operation for designating any of the plurality of defective parts, performed by the operator, the input interface 120 outputs an electrical signal corresponding to the input operation to the processing circuitry 140. The acquisition function 141 acquires designation information of the defective part designated by the operator by receiving the electrical signal output from the input interface 120.

When the detection function 144 detects a defective part, the time identification function 145 acquires time information added to the frame of the contrast-enhanced image in which the defective part is first detected (hereinafter referred to as a defect detection frame). The time identification function 145 identifies the time when the image of the defect detection frame was captured (hereinafter referred to as a defect start time) on the basis of the acquired time information. The time identification function 145 is an example of a time identificator. The display control function 143 causes the display 130 to display the defect start time identified by the time identification function 145.

The boundary setting function 146 performs segmentation for setting a boundary line between a defective part and a peripheral part in a contrast-enhanced image detected by the detection function 144. The boundary setting function 146 sets an ROI including a defective part and the peripheral part thereof in a defect detection frame in segmentation. The ROI may be set arbitrarily and, for example, is set in a square shape in which the area ratio of the defective part and the peripheral portion is equal.

The boundary setting function 146 sets a boundary between the defective part and the peripheral part in the set ROI. The defect degree determination function 147 determines the degree of defect in the defective part on the basis of an intensity difference between the defective part and the peripheral part in the contrast-enhanced image segmented by the boundary setting function 146. The intensity difference between the defective part and the peripheral part appears as a luminance difference in the contrast-enhanced image. The boundary setting function 146 is an example of a boundary setter, and the peripheral part is an example of a non-defective part.

The defect degree determination function 147 determines marked washout with a high degree of washout when the average intensity of the peripheral part and the average intensity of the defect part satisfy the following formula (1), for example. Furthermore, when the average intensity of the defective part satisfies the following formula (2), the defect degree determination function 147 determines mild washout with a moderate degree of washout. The defect degree determination function 147 is an example of a defect degree determiner.

Average intensity of defect part/average intensity of peripheral part < first threshold (1)

First threshold $\leq$ average intensity of defect part/average intensity of peripheral part < second threshold (2)

The time curve generation function 148 motion-compensates for a contrast-enhanced image in the artery phase in the reverse time direction from the portal vein phase using, for example, information on the detection ROI in tissue images (hereinafter referred to as detection ROI information), and generates a time intensity curve (hereinafter, TIC). The TIC indicates temporal change in stain (echo signal intensity) of the contrast medium in a lesioned part and is obtained from temporal change in the luminance of the lesioned part in contrast-enhanced images. FIG. 4 is a diagram schematically showing a contrast-enhanced image and a tissue image. The position of a defective part image GA31 in a contrast-enhanced image GA30 corresponds to the position of a detection ROI GA36 in a tissue image GA35.

The time curve generation function 148 acquires a TIC of the lesioned part by performing motion compensation in the reverse time direction using the detection ROI GA36 in the tissue image GA35. FIG. 5 is a diagram showing an example of a TIC. A solid line L1 indicates temporal change in the enhancement intensity of a contrast medium in a lesion. A dashed line L2 indicates temporal change in the enhancement intensity of the contrast medium around a defect. The time curve generation function 148 is an example of a time curve generator.

The peak frame selection function 149 selects a peak frame in which enhancement of the contrast medium reaches a peak on the basis of the TIC generated by the time curve generation function 148. The peak frame selection function 149 obtains a peak time T1 at which enhancement of the contrast medium in the lesion reaches a peak with reference to the TIC. The peak frame selection function 149 selects a contrast-enhanced image at the peak time T1 as the peak frame. The peak frame selection function 149 stores the selected peak frame in the memory 180. The display control function 143 causes the display 130 to display the peak frame selected by the peak frame selection function 149 along with the tissue image at the peak time T1. The peak frame selection function 149 is an example of a peak frame selector.

The finding creation function 150 creates findings of enhancement of an arterial phase on the basis of the signal intensity difference, for example, the luminance difference, between a defective part and the peripheral part in the peak frame selected by the peak frame selection function 149. For example, findings are created as "high" when that a case where enhancement of a lesioned part is greater than that of the peripheral part, "iso" when enhancement of the lesioned part and enhancement of the peripheral part are similar, and "low" when enhancement of the lesioned part is less than that of the peripheral part. The finding creation function 150 stores the created findings of arterial enhancement in the memory 180. The finding creation function 150 is an example of a finding creator.

Next, an example of processing of the medical image processing device 100 will be described. First, the medical image processing device 100 examines a subject to acquires examination data and acquires examination results on the basis of the acquired examination data. For this reason, processing at the time of acquiring examination data will be described first, and then processing using the acquired examination data will be described.

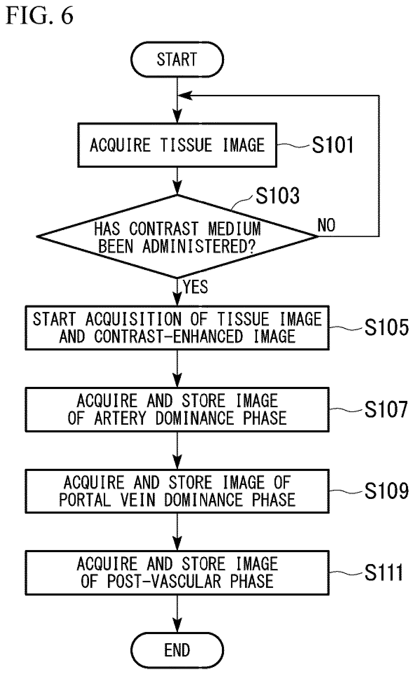

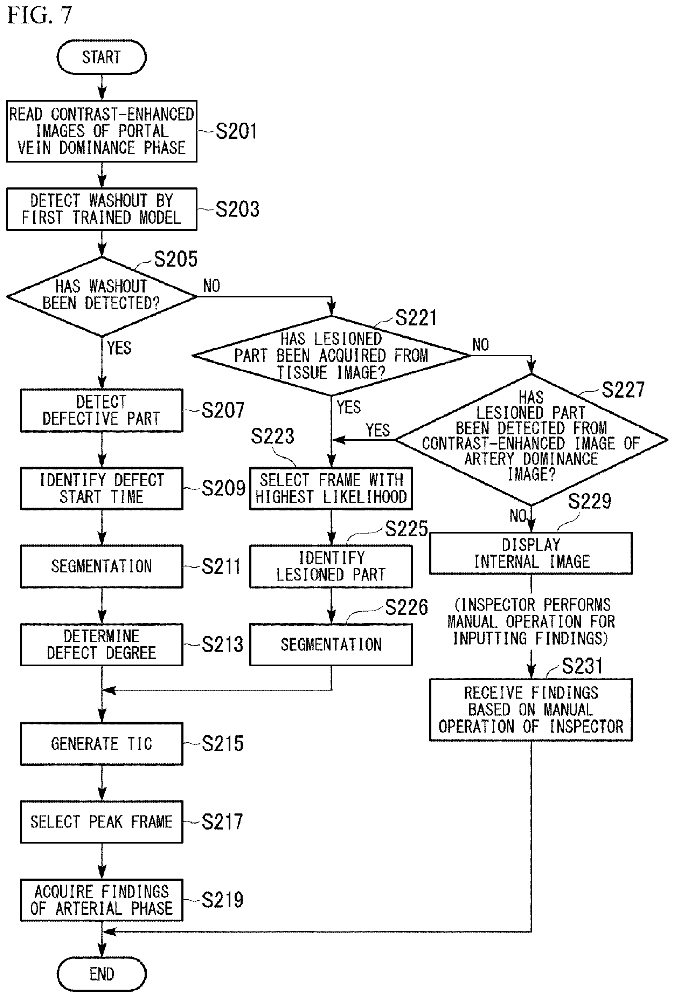

FIG. 6 and FIG. 7 are flowcharts showing an example of processing of the medical image processing device 100. FIG. 6 shows processing at the time of acquiring examination data. When an operator operates the ultrasonic probe 10 in the ultrasonic diagnostic apparatus 1, the ultrasonic probe 10 transmits echo data to the medical image processing device 100. The medical image processing device 100 receives the echo data transmitted by the ultrasonic probe 10 through the acquisition function 141. The image generation function 142 generates and acquires a tissue image on the basis of echo data of tissue images acquired by the acquisition function 141 (step S101) and stores the tissue image in the memory 180.

Subsequently, the image generation function 142 determines whether or not a contrast medium has been administered to a subject on the basis of whether or not an electrical signal corresponding to administration information has been output from the input interface 120 (step S103). If it is determined that the contrast medium has not been administered to the subject, the image generation function 142 returns processing to step S101 and acquires the tissue image.

If it is determined that the contrast medium has been administered to the subject, the image generation function 142 acquires the tissue image and starts acquisition of a contrast-enhanced image on the basis of echo data of the contrast medium acquired by the acquisition function 141 (step S105). Subsequently, while a phase after administration of the contrast medium is an artery dominant phase, the image generation function 142 acquires an internal image of the artery dominant phase and stores the internal image in the memory 180 (step S107).

While the phase after administration of the contrast medium is a portal vein dominant phase, the image generation function 142 acquires an internal image of the portal vein dominant phase and stores the internal image in the memory 180 (step S109). While the phase after administration of the contrast medium is a post-vascular phase, the image generation function 142 acquires an internal image of the post-vascular phase and stores the internal image in the memory 180 (step S111). Accordingly, the medical image processing device 100 ends the processing shown in FIG. 6. After the processing shown in FIG. 6 ends, a plurality of tissue images before administration of the contrast medium, a plurality of tissue images and contrast-enhanced images after administration of the contrast medium are stored in the memory 180.

Next, processing at the time of acquiring examination results on the basis of acquired examination data. FIG. 7 shows processing at the time of acquiring examination results on the basis of examination data. In the medical image processing device 100, the detection function 144 reads contrast-enhanced images after a portal vein dominant phase stored in the memory 180 (step S201). Subsequently, the detection function 144 detects washout on the basis of output results obtained by inputting the read contrast-enhanced images after the portal vein dominant phase to the first trained model 184 (step S203).

The detection function 144 determines whether or not washout has been detected (step S205). If it is determined that washout has been detected, the detection function 144 detects a defective part in the contrast-enhanced images (step S207). Subsequently, the time identification function 145 identifies a defect start time (step S209), and the display control function 143 causes the display 130 to display the defect start time. The defect start time displayed on the display control function 143 is automatically input as findings or added to findings by an input operation performed on the input interface 120 by the operator, for example.

Subsequently, the boundary setting function 146 performs segmentation on a defect detection frame in which the defective part is first detected by the detection function 144 (step S211) and identifies the defect part and the peripheral part. Subsequently, the defect degree determination function 147 determines a defect degree of the defective part in the segmented defect detection frame (step S213).

Subsequently, the time curve generation function 148 performs motion compensation due to respiratory fluctuations and the like using detection ROI information in tissue images, and generates a TIC of a lesioned part in the contrast-enhanced images (step S215). Subsequently, the peak frame selection function 149 selects a peak frame on the basis of the TIC generated by the time curve generation function 148 (step S217). Subsequently, the finding creation function 150 creates findings of enhancement of an arterial phase on the basis of the luminance difference between the lesioned part and the peripheral part in the peak frame selected by the peak frame selection function 149 (step S219). Accordingly, the medical image processing device 100 ends the processing shown in FIG. 7.

If it is determined in step S205 that washout has not been detected, the detection function 144 detects the presence or absence of a lesioned part on the basis of results obtained by inputting tissue images of the artery dominant phase to the second trained model 185. As a result, the detection function 144 determines whether or not a lesioned part has been detected (step S221).

If it is determined that a lesioned part has been detected, the detection function 144 identifies the lesioned part and selects a frame with a highest likelihood from among a plurality of contrast-enhanced image frames (step S223). Subsequently, the detection function 144 identifies the position of the lesioned part in the contrast-enhanced images on the basis of the position of the lesioned part in the tissue images by performing motion compensation in the time direction and the reverse time direction, starting from the selected frame (step S225). Thereafter, the medical image processing device 100 segments the contrast-enhanced images (step S226) and advances processing to step S215.

If it is determined in step S221 that no lesioned part has been detected, the detection function 144 determines whether or not a lesioned part has been detected from the contrast-enhanced images of the artery dominant phase (step S227). If the detection function 144 determines that a lesioned part has been detected, the medical image processing device 100 advances processing to step S223.

When the detection function 144 determines that no defective part has been detected, the display control function 143 causes the display 130 to display an internal image (step S229). The operator who views the internal image displayed on the display 130 identifies the defective part (lesioned part) on the basis of the internal image and performs a manual operation for inputting findings on the input interface 120. The input interface 120 outputs an electrical signal corresponding to the manual operation to the processing circuitry 140. The acquisition function 141 receives an input based on the manual operation of the operator (step S231). Accordingly, the medical image processing device 100 ends the processing shown in FIG. 7.

In the process of executing an examination by the medical image processing device 100, the medical image processing device 100 simultaneously performs processing for updating the first trained model 184 to the third trained model 186 on the basis of input data. Thereamong, processing for updating the first trained model 184 will be described below.

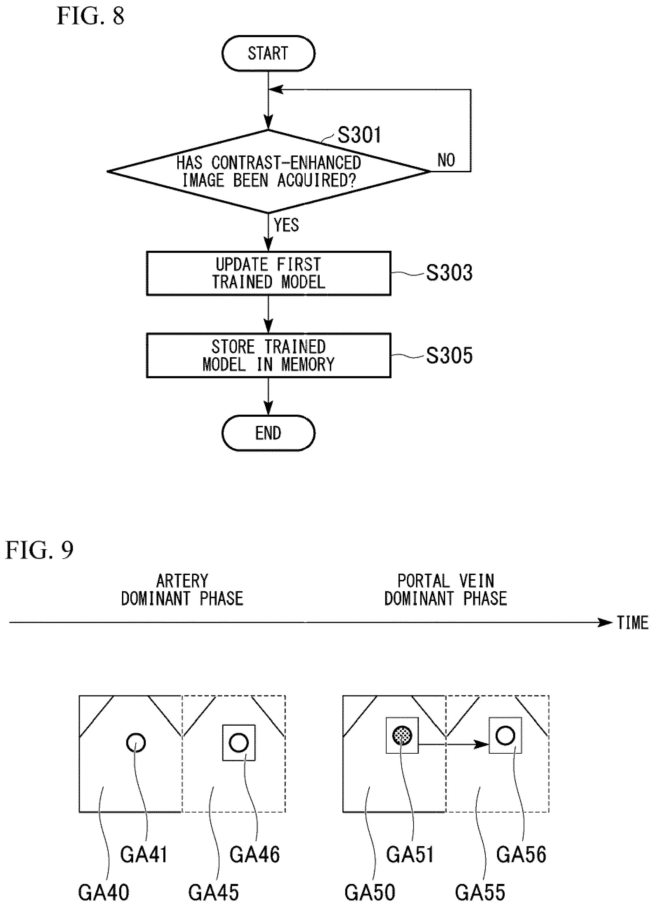

FIG. 8 is a flowchart showing an example of processing for updating the first trained model 184. The medical image processing device 100 determines whether or not the acquisition function 141 has acquired a contrast-enhanced image after a portal vein dominant phase (step S301). If it is determined that the acquisition function 141 has not acquired a contrast-enhanced image after the portal vein dominant phase, the medical image processing device 100 returns processing to step S301.

If it is determined that the acquisition function 141 has acquired a contrast-enhanced image after the portal vein dominant phase, the medical image processing device 100 updates the first trained model 184 using the first trained model 184 stored in the memory 180 and the acquired contrast-enhanced image (step S303). Subsequently, the medical image processing device 100 stores the updated first trained model 184 in the memory 180 (step S305). Accordingly, the medical image processing device 100 ends the processing shown in FIG. 8. The medical image processing device 100 also updates the second trained model 185 and the third trained model 186 through the same procedure.

The medical image processing device 100 of the first embodiment detects washout on the basis of contrast-enhanced images after the portal vein dominant phase. After the portal vein dominant phase, change in the contrast-enhanced images is less than that in the artery dominant phase. As a result, washout is easily detected, and thus effort for selecting an appropriate defect detection frame, a peak frame, and the like can be reduced and lesions can be detected appropriately.

(Second embodiment)

Next, a second embodiment will be described. Although internal images are continuously captured after administration of a contrast medium to a subject in the first embodiment, a contrast-enhanced image of an artery dominant phase and a contrast-enhanced image of a portal vein dominant phase are independently captured in the second embodiment. In this respect, the second embodiment is mainly different from the first embodiment.

When an artery dominant phase and a portal vein dominant phase have been independently captured, and a defective part (lesioned part) has been detected by detecting washout in the portal vein dominant phase, it is difficult to identify a lesioned part in the artery dominance phase. Therefore, in the second embodiment, the detection function 144 searches arbitrary tissue image frames in the later stage of the artery dominance phase for a position pattern corresponding to the defective part in frames in tissue images in a time period in which a defect detection frame was captured. The detection function 144 detects a lesioned part in a contrast-enhanced image of the artery dominant phase on the basis of the movement of the searched pattern. Specifically, the detection function 144 searches for a site best matching the searched pattern. The detection function 144 detects a lesioned part by identifying a site of the contrast-enhanced image corresponding to the searched site of the tissue images in the artery dominant phase as the lesioned part.

FIG. 9 is a diagram schematically showing internal images in an artery dominant phase and internal images in a portal vein dominant phase according to the second embodiment. FIG. 9 shows a contrast-enhanced image GA40 and a tissue image GA45 as internal images in the artery dominant phase, and a contrast-enhanced image GA50 and a tissue image GA55 as internal images in the portal vein dominant phase.

The detection function 144 extracts a pattern of a corresponding position image GA56 in the tissue image GA55, which corresponds to a defective part image GA51 in the contrast-enhanced image GA50 in the portal vein dominant phase, for example. Subsequently, the detection function 144 searches for a corresponding position image GA46 having a pattern best matching the pattern of the corresponding position image GA56 of the tissue image GA55 in the portal vein dominant phase in the tissue image GA45 in the artery dominant phase. Upon searching for the corresponding position image GA46, the detection function 144 identifies the portion of the contrast-enhanced image GA40 corresponding to the corresponding position image GA46 as a defective part image GA41.

Although moving images of the artery dominant phase and the portal vein dominant phase are captured independently, and the moving images of the artery dominant phase and the portal vein dominant phase are discontinuous (not continuous) in the second embodiment, even if the moving images of the artery dominant phase and the portal vein dominant phase are discontinuous, a defective part detected in the portal vein dominant phase can be identified in a contrast-enhanced image in the artery dominant phase as in the first embodiment.

In the second embodiment, the medical image processing device 100 detects a position corresponding to the corresponding position image GA46 having a pattern best matching the pattern of the corresponding position image GA56 of the tissue image GA55 in the portal vein dominant phase as a lesioned part in the artery dominant phase. On the other hand, the medical image processing device 100 may identify a lesioned part in the artery dominance phase, for example, through manual correction of motion-compensating for the tissue image GA55 of the portal vein dominant phase in the reverse time direction.

(Third Embodiment)

Next, a third embodiment will be described. Although internal images are captured up to a post-vascular phase during an examination, and then a defect part is identified and findings are created in the first and second embodiments, a defective part is identified and findings are created simultaneously with an examination in the third embodiment. In this respect, the third embodiment mainly differs from the first and second embodiments.

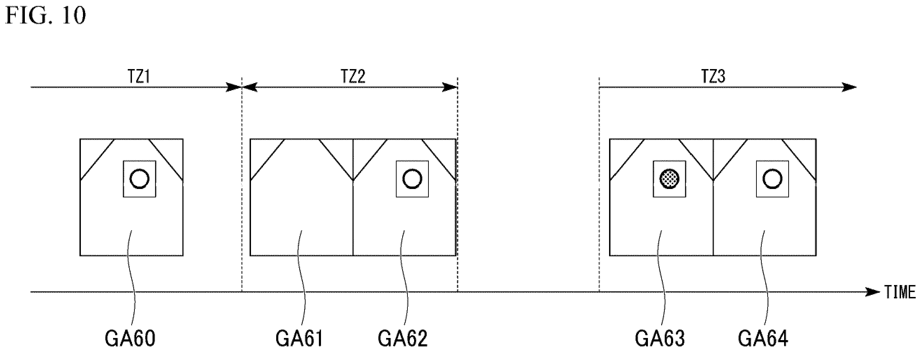

FIG. 10 is a diagram schematically showing a tissue image before administration of a contrast medium, internal images in an artery dominant phase and a portal vein dominant phase in the third embodiment. In the ultrasonic diagnostic apparatus 1, an operator operates the ultrasonic probe 10 to examine a subject. While the subject is being examined, the medical image processing device 100 receives echo data output by the ultrasonic probe 10.

In a first time period TZ1 before administration of the contrast medium to the subject, the medical image processing device 100 generates a tissue image GA60 on the basis of echo data of tissue images output by the ultrasonic probe 10 through the image generation function 142. The display control function 143 causes the display 130 to display the tissue image GA60 generated by the image generation function 142 on the basis of the echo data of the tissue images output by the ultrasonic probe 10.

As the examination progresses, a second time zone TZ2 immediately after administration of the contrast medium to the subject becomes an artery dominant phase, and the medical image processing device 100 generates a contrast-enhanced image GA61 through the image generation function 142 on the basis of the echo data output by the ultrasonic probe 10. The contrast-enhanced image GA61 generated here is a contrast-enhanced image in the artery dominant phase. Furthermore, the image generation function 142 generates a tissue image GA62 on the basis of the echo data of the tissue images output by the ultrasonic probe 10.

As the examination further progresses, a third time period TZ3 after passing the artery dominant phase becomes a portal vein dominant phase, and the medical image processing device 100 performs generates a contrast-enhanced image GA63 on the basis of the echo data output by the ultrasonic probe 10 through the image generation function 142 as in the second time period TZ2. The contrast-enhanced image GA63 generated here is a contrast-enhanced image in the portal vein dominant phase. Furthermore, the image generation function 142 generates a tissue image GA64 on the basis of the echo data of the tissue images output by the ultrasonic probe 10.

In the third embodiment, the medical image processing device 100 sets a detection ROI in the tissue image GA60 in the first time period TZ1 before administration of the contrast medium to the subject. Furthermore, the medical image processing device 100 detects the presence or absence of a lesion through the detection function 144 on the basis of output results obtained by inputting the tissue image GA60 generated by the image generation function 142 to the second trained model 185.

In the second time period TZ2 following the first time period TZ1, the medical image processing device 100 detects the presence or absence of a lesioned part on the basis of output results obtained by inputting the contrast-enhanced image GA61 of the artery dominance phase generated by the image generation function 142 to the third trained model 186 through the detection function 144. When the detection function 144 has detected a lesioned part, the time curve generation function 148 generates a TIC and the peak frame selection function 149 selects a peak frame. Then, the finding creation function 150 creates findings on the basis of the peak frames selected by the peak frame selection function 149.

In the third time period TZ3 following the second time period TZ2, the medical image processing device 100 detects the presence or absence of a defective part on the basis of output results obtained by inputting the contrast-enhanced image GA63 of the portal vein dominance phase generated by the image generation function 142 into the first trained model 184 through the detection function 144. When the detection function 144 has detected a defective part in the contrast-enhanced image GA63, the time identification function 145 acquires a defect start time.

After the detection function 144 detects a defective part in the second time period TZ2 or the third time period TZ3, the boundary setting function 146 performs segmentation for setting a boundary between the defective part and the peripheral part, and the defect degree determination function 147 determines a defect degree of the defective part. Thereafter, when a lesion cannot be detected in the artery dominant phase, the time curve generation function 148 generates a TIC, and the peak frame selection function 149 selects a peak frame. Then, the finding creation function 150 creates findings on the basis of the peak frames selected by the peak frame selection function 149.

The medical image processing device of the third embodiment has the same effects as the medical image processing device of the first embodiment. Furthermore, the medical image processing device of the third embodiment can detect a lesion simultaneously with an examination of a subject.

(Fourth embodiment)

Next, a fourth embodiment will be described. The fourth embodiment differs in that each function incorporated in the processing circuitry 140 of the ultrasonic diagnostic apparatus 1 in the first embodiment is incorporated into a medical information processing device 300. Therefore, the following description will focus on differences from the first embodiment, and the description of the points that are common to the first embodiment will be omitted. In the description of the fourth embodiment, the same parts as those in the first embodiment are denoted by the same reference numerals.

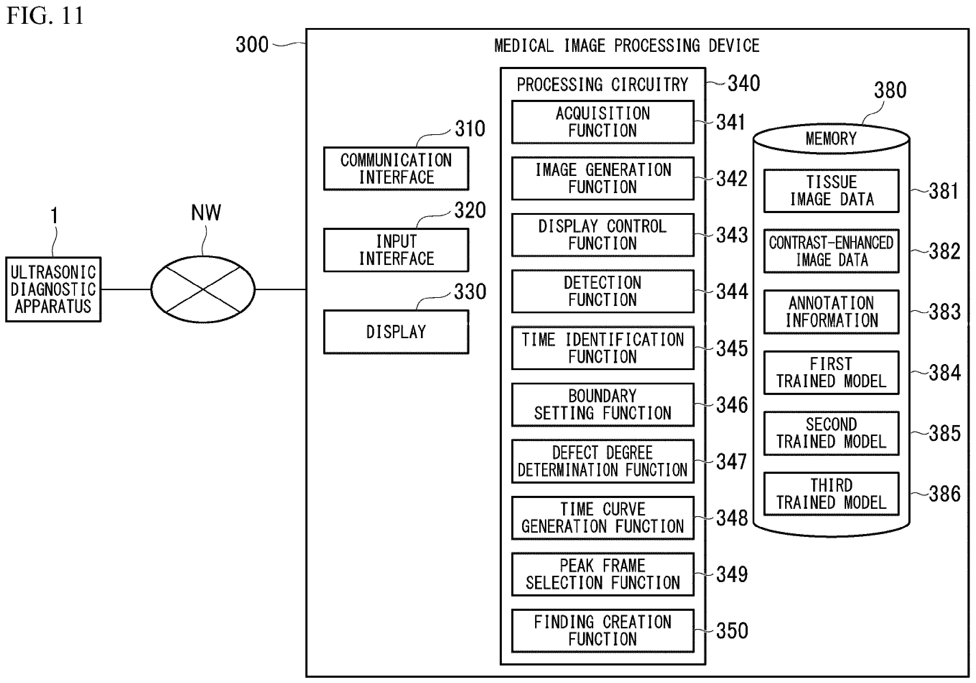

FIG. 11 is a block diagram showing an example of a configuration of the medical information processing device 300 of the fourth embodiment. The medical information processing device 300 receives a plurality of internal images captured by the ultrasonic diagnostic apparatus 1 via a communication network NW. The medical information processing device 300 includes, for example, a communication interface 310, an input interface 320, a display 330, processing circuitry 340, and a memory 380.

The communication interface 310 communicates with an external device such as the ultrasonic diagnostic apparatus 1 via the communication network NW. The communication interface 310 includes, for example, a communication interface such as an NIC.

The input interface 320 receives various input operations from an operator of the medical information processing device 300, converts the received input operations into electrical signals, and outputs the electrical signals to the processing circuitry 340. For example, the input interface 320 includes a mouse, a keyboard, a trackball, a switch, a button, a joystick, a touch panel, and the like. The input interface 320 may be, for example, a user interface that receives voice input, such as a microphone. When the input interface 320 is a touch panel, the input interface 320 may also have the display function of the display 330.

It should be noted that the input interface in the present specification is not limited to those having physical operation parts such as a mouse and a keyboard. For example, examples of the input interface include an electrical signal processing circuit that receives an electrical signal corresponding to an input operation from external input equipment provided separately from the device and outputs the electrical signal to a control circuit.

The display 330 displays various types of information. For example, the display 330 displays images generated by the processing circuitry 340, a graphical user interface (GUI) for receiving various input operations from the operator, and the like. For example, the display 330 is an LCD, a CRT display, an organic EL display, or the like.

The processing circuitry 340 includes, for example, an acquisition function 341, a display control function 343, a detection function 344, a time identification function 345, a boundary setting function 346, a defect degree determination function 347, a time curve generation function 348, a peak frame selection function 349, and a finding creation function 350. The processing circuitry 340 realizes these functions by, for example, a hardware processor (computer) executing a program stored in the memory 380 (storage circuit).

The hardware processor refers to, for example, a circuitry such as a CPU, a GPU, an application specific integrated circuit (ASIC), a programmable logic device (for example, a simple programmable logic device (SPLD), a complex programmable logic device (CPLD), or a field programmable gate array (FPGA)) or the like. Instead of storing the program in the memory 380, the program may be directly embedded in the circuitry of the hardware processor. In this case, the hardware processor realizes the functions by reading and executing the program embedded in the circuitry. The aforementioned program may be stored in the memory 380 in advance, or may be stored in a non-transitory storage medium such as a DVD or CD-ROM and installed in the memory 380 from the non-transitory storage medium when the non-transitory storage medium is set in a drive device (not shown) of the medical information processing device 300. The hardware processor is not limited to being configured as a single circuit and may be configured as one hardware processor by combining a plurality of independent circuits to realize each function. Further, a plurality of components may be integrated into one hardware processor to realize each function.

The acquisition function 341 acquires information on internal images transmitted from the ultrasonic diagnostic apparatus 1. The information on the internal images includes tissue image data 381 and contrast-enhanced image data 382. The acquisition function 341 stores the acquired tissue image data 381 and contrast-enhanced image data 382 in the memory 380.

The display control function 343 causes the display 330 to display various images such as tissue images and contrast-enhanced images based on the tissue image data 381 and contrast-enhanced image data 382 stored in the memory 380. The detection function 344 has the same function as that of the detection function 144 of the first embodiment. The detection function 344 detects the presence or absence of a defective part, for example, on the basis of contrast-enhanced images after a portal vein dominant phase.

The time identification function 345, the boundary setting function 346, the defect degree determination function 347, the time curve generation function 348, the peak frame selection function 349, and the finding creation function 350 have the same functions as those of the time identification function 145 and the boundary setting function 146, the defect degree determination function 147, the time curve generation function 148, the peak frame selection function 149, and the finding creation function 150 of the first embodiment.

According to the fourth embodiment described above, the same effects as those of the first embodiment are obtained. Furthermore, in the fourth embodiment, effort for selecting an appropriate frame can be reduced, and lesions can be detected appropriately. Moreover, detection of defects and creation of findings can be collectively executed in a plurality of ultrasonic diagnostic apparatuses 1. Although the acquisition function 341 acquires echo data and imaging data transmitted by the ultrasonic diagnostic apparatus 1 and the image generation function 342 generates and acquires contrast-enhanced image on the basis of the echo data and the imaging data in the medical information processing device 300 in the fourth embodiment, the acquisition function 341 may generate and acquire contrast-enhanced images on the basis of data provided by a modality other than the ultrasonic diagnostic apparatus 1.

In the above-described first embodiment, the medical image processing device 100 detects and generates data and the like used for detecting a defective part. On the other hand, for example, correction instruction information with respect to data used for detecting a defective part output by the input interface 120 when the operator operates the input interface 120 may be acquired by the acquisition function 141, and the data used for detecting a defective part may be corrected on the basis of the acquired information. Moreover, elements of the first to fourth embodiments may be combined or replaced as appropriate.

According to at least one embodiment described above, it is possible to reduce effort for selecting an appropriate frame to appropriately detect a lesion by having an acquirer that acquires a contrast-enhanced image of a subject at least after a portal vein dominant phase, among contrast-enhanced images of the subject to which a contrast medium has been administered in a process of reaching a post-vascular phase from an artery dominant phase via the portal vein dominant phase, and a detector that detects a site where the contrast medium has been washed out as a defective part in the contrast-enhanced image after the portal vein dominant phase.

Although several embodiments have been described, these embodiments are presented as examples and are not intended to limit the scope of the invention. These embodiments can be implemented in various other forms, and various omissions, substitutions, and modifications can be made without departing from the scope of the invention as defined by the appended claims.

**Claims**

1.  A medical image processing device (100) comprising:

    an acquirer (141, 341) configured to acquire contrast-enhanced images of a subject to which a contrast medium has been administered in a process of reaching a post-vascular phase from an artery dominant phase via a portal vein dominant phase, the acquirer being configured to acquire contrast enhanced images at least after the portal vein dominant phase has been reached, the contrast enhanced images being generated by an ultrasound diagnostic apparatus (1);
    a detector (144) configured to detect a site where the contrast medium has been washed out as a lesioned part in the contrast-enhanced images after the portal vein dominant phase;
    a time identificator (145) configured to identify a time when a detection frame, which is a frame of the contrast-enhanced images in which the lesioned part is detected among the plurality of contrast-enhanced images, was captured;
    **characterised in that** the medical image processing device (100) further comprises:

a boundary setter (146) configured to set a boundary between the lesioned part and a non-lesioned part in the detection frame by performing segmentation in the detection frame;

a defect degree determiner (147) configured to determine a degree of washout in the lesioned part on the basis of a luminance difference between the lesioned part and the non-lesioned part in the detection frame;

a time curve generator (148) configured to generate a time intensity curve indicating temporal change in enhancement of the contrast medium in the lesioned part by motion-compensating for a contrast-enhanced image in an artery phase in the reverse time direction from a portal vein phase using a region-of-interest including the lesioned part and the non-lesioned part;

a peak frame selector (149) configured to select a peak frame in which the enhancement of the contrast medium reaches a peak in the lesioned part on the basis of the time intensity curve;

a finding creator (150, 350) configured to create findings of the enhancement of the contrast medium in the peak frame on the basis of the luminance difference between the lesioned part and the non-lesioned part in the peak frame.

2. The medical image processing device (100) according to claim 1, wherein the detector (144, 344) is configured to detect the lesioned part on the basis of output results obtained by inputting the contrast-enhanced images of the subject to a first trained model (184, 384) generated by learning the contrast-enhanced images including the lesioned part as training data.

3. The medical image processing device (100) according to any one of claims 1 to 2, wherein the portal vein dominant phase is defined on the basis of time information measured by a timer or annotation information set for an examination process.

4. The medical image processing device (100) according to any one of claims 1 to 3, wherein the acquirer (141, 341) is further configured to acquire designation information of a lesioned part designated by an operator when the detector (144, 344) detects a plurality of lesioned parts.

5. The medical image processing device (100) according to any one of claims 1 to 4, wherein the acquirer (141, 341) is further configured to acquire a tissue image of the subject captured along with the contrast-enhanced images, and the detector (144, 344) is configured to search for a tissue image pattern at a position corresponding to the lesioned part in the contrast-enhanced images and detect a best matching portion as the lesioned part in the contrast-enhanced images of the artery dominance phase.

6. The medical image processing device (100) according to claim 1, wherein the acquirer (141, 341) is further configured to acquire a tissue image of the subject captured along with the contrast-enhanced images, and the detector (144, 344) is configured to:

detect the lesioned part in the tissue image in the artery dominant phase when the lesioned part in the contrast-enhanced images in the portal vein dominant phase has not been detected; and

select a frame with a highest likelihood of the lesioned part from among the frames of the contrast-enhanced images on the basis of the tissue image in the artery dominant phase in which the lesioned parts is detected, further comprising:

a boundary setter (146) configured to set a boundary between the lesioned part and a non-lesioned part in the frame with the highest likelihood of the lesioned part by performing segmentation in the frame with the highest likelihood of the lesioned part;

a time curve generator (148, 348) configured to perform motion compensation in a reverse time direction starting from the frame with the highest likelihood using a region-of-interest including the lesioned part and the non-lesioned part to generate a time intensity curve indicating temporal change in the enhancement of the contrast medium in the lesioned part;

a peak frame selector (149, 349) configured to select a peak frame in which the enhancement of the contrast medium reaches a peak in the lesioned part on the basis of the time intensity curve; and

a finding creator (150, 350) configured to create findings of the enhancement of the contrast medium in the peak frame on the basis of the luminance difference between the lesioned part and the non-lesioned part in the peak frame.

7. The medical image processing device (100) according to claim 6, wherein the detector (144, 344) is configured to identify the lesioned part on the basis of output results obtained by inputting the tissue image into a second trained model (185, 385) generated by using the tissue image including the lesioned part as training data.

8. The medical image processing device (100) according to any one of claims 1 to 7, wherein the acquirer (141, 341) is further configured to acquire a contrast-enhanced image of the subject in the artery dominant phase, and the detector (144, 344) is configured to detect a lesioned part from the contrast-enhanced images of the artery dominant phase.

9. The medical image processing device (100) according to claim 8, wherein the detector (144, 344) is configured to detect the lesioned part on the basis of output results obtained by inputting the contrast-enhanced images of the artery dominant phase into a third trained model (186, 386) generated by using the contrast-enhanced images of the artery dominant phase including the lesioned part as training data.

10. The medical image processing device (100) according to any one of claims 1 to 9, wherein the acquirer (141, 341) is configured to acquire correction instruction information for data used to detect the lesioned part.

11. The medical image processing device (100) according to claim 1, wherein the acquirer (141, 341) is configured to acquire a tissue image of the subject captured before administration of the contrast medium to the subject and a contrast-enhanced image of the subject to which the contrast medium has been administered, and the detector is configured to detect a lesioned part in which the contrast medium has been washed out in the tissue image of the subject, the contrast-enhanced image of the artery dominant phase, and contrast-enhanced images after the portal vein dominant phase.

12. An ultrasonic diagnostic apparatus (1) comprising:

the medical image processing device (100) according to any one of claims 1 to 11; and
an ultrasonic probe (10) configured to transmit ultrasonic waves and receive echo of the transmitted ultrasonic waves,
wherein the acquirer (141, 341) is configured to acquire the contrast-enhanced images generated on the basis of ultrasonic echo received by the ultrasonic probe (10).

13. The medical image processing device (100) according to any one of claims 1 to 11, wherein the acquirer (141, 341) is configured to acquire the contrast-enhanced images provided by a modality connected via a network.

14. A computer-readable storage medium storing a program causing, when said program is executed by a computer, the computer to:

acquire contrast-enhanced images of a subject at least after a portal vein dominant phase has been reached, the said contrast-enhanced images being among contrast-enhanced images of the subject to which a contrast medium has been administered in a process of reaching a post-vascular phase from an artery dominant phase via the portal vein dominant phase, the contrast-enhanced images being generated by an ultrasound diagnostic apparatus (1);
detect a site where the contrast medium has been washed out as a lesioned part in the contrast-enhanced images after the portal vein dominant phase;
identify a time when a detection frame, which is a frame of the contrast-enhanced images in which the lesioned part is detected among the plurality of contrast-enhanced images was captured;
**characterised in that** the computer is further caused to:

set a boundary between the lesioned part and a non-lesioned part in the detection frame by performing segmentation in the detection frame;
determine a degree of washout in the lesioned part on the basis of a luminance difference between the lesioned part and the non-lesioned part in the detection frame;
generate a time intensity curve indicating temporal change in enhancement of the contrast medium in the lesioned part by motion-compensating for a contrast-enhanced image in an artery phase in the reverse time direction from a portal vein phase using a region-of-interest including the lesioned part and the non-lesioned part;
select a peak frame in which the enhancement of the contrast medium reaches a peak in the lesioned part on the basis of the time intensity curve;
create findings of the enhancement of the contrast medium in the peak frame on the basis of the luminance difference between the lesioned part and the non-lesioned part in the peak frame.

**Patentansprüche**

1. Medizinische Bildverarbeitungsvorrichtung (100), umfassend:

    ein Erfassungsgerät (141, 341), das so konfiguriert ist, dass es kontrastverstärkte Bilder eines Probanden erfasst, dem ein Kontrastmittel verabreicht wurde, während er in einem Prozess von einer arteriell dominanten Phase über eine portalvenös dominanten Phase eine postvaskuläre Phase erreicht, wobei das Erfassungsgerät so konfiguriert ist, dass es kontrastverstärkte Bilder mindestens nach Erreichen der portalvenös dominanten Phase erfasst, wobei die kontrastverstärkten Bilder von einer Ultraschall-Diagnoseeinrichtung (1) erzeugt werden;
    einen Detektor (144), der so konfiguriert ist, dass er eine Stelle, an der das Kontrastmittel ausgewaschen wurde, als einen lädierten Teil in den kontrastverstärkten Bildern nach der portalvenösen dominanten Phase detektiert;
    einen Zeitidentifikator (145), der so konfiguriert ist, dass er einen Zeitpunkt identifiziert, zu dem ein Detektionseinzelbild aufgenommen wurde, das ein Einzelbild der kontrastverstärkten Bilder ist, in dem der lädierte Teil unter der Vielzahl von kontrastverstärkten Bildern detektiert wurde;
    **dadurch gekennzeichnet, dass** die medizinische Bildverarbeitungsvorrichtung (100) weiter umfasst:

       einen Grenzsetzer (146), der so konfiguriert ist, dass er eine Grenze zwischen dem lädierten Teil und einem nicht lädierten Teil im Detektionseinzelbild durch Durchführen einer Segmentierung im Detektionseinzelbild festlegt;
       ein Defektgradbestimmungsgerät (147), das so konfiguriert ist, dass es den Auswaschungsgrad im lädierten Teil basierend auf einer Luminanzdifferenz zwischen dem lädierten Teil und dem nicht lädierten Teil im Detektionseinzelbild bestimmt;
       einen Zeitkurvengenerator (148), der so konfiguriert ist, dass er eine Zeit-Intensitäts-Kurve erzeugt, die eine zeitliche Veränderung der Kontrastmittelanreicherung im lädierten Teil anzeigt, indem ein kontrastverstärktes Bild in einer Arterienphase in umgekehrter Zeitrichtung aus einer Portalvenenphase unter Verwendung einer Region von Interesse, die den lädierten Teil und den nicht lädierten Teil beinhaltet, bewegungskompensiert wird;
       ein Peak-Einzelbild-Auswahlgerät (149), das so konfiguriert ist, dass es ein Peak-Einzelbild, in dem die Kontrastmittelanreicherung im lädierten Teil den Höhepunkt erreicht, basierend auf der Zeit-Intensitäts-Kurve auswählt;
       einen Befundgenerator (150, 350), der so konfiguriert ist, dass er Befunde der Kontrastmittelanreicherung im Peak-Einzelbild basierend auf der Luminanzdifferenz zwischen dem lädierten Teil und dem nicht lädierten Teil im Peak-Einzelbild erstellt.

2. Medizinische Bildverarbeitungsvorrichtung (100) nach Anspruch 1, wobei der Detektor (144, 344) so konfiguriert ist, dass er den lädierten Teil basierend auf Ausgabeergebnissen detektiert, die durch Eingabe der kontrastverstärkten Bilder des Probanden in ein erstes trainiertes Modell (184, 384) erhalten werden, das durch Lernen der kontrastverstärkten Bilder, die den lädierten Teil beinhalten, als Trainingsdaten erzeugt wurde.

3. Medizinische Bildverarbeitungsvorrichtung (100) nach einem der Ansprüche 1 oder 2, wobei die portalvenös dominante Phase basierend auf Zeitinformationen, die mit einem Zeitgeber gemessen werden, oder Annotationsinformationen, die für einen Untersuchungsprozess festgelegt sind, definiert wird.

4. Medizinische Bildverarbeitungsvorrichtung (100) nach einem der Ansprüche 1 bis 3, wobei das Erfassungsgerät (141, 341) weiter so konfiguriert ist, dass es Bezeichnungsinformationen eines vom Bediener bezeichneten lädierten Teils erfasst, wenn der Detektor (144, 344) eine Vielzahl lädierter Teile detektiert.

5. Medizinische Bildverarbeitungsvorrichtung (100) nach einem der Ansprüche 1 bis 4, wobei das Erfassungsgerät (141, 341) weiter so konfiguriert ist, dass es ein Gewebebild des Probanden, das zusammen mit den kontrastverstärkten Bildern aufgenommen wurde, erfasst, und der Detektor (144, 344) so konfiguriert ist, dass er in den kontrastverstärkten Bildern an einer Position, die dem lädierten Teil entspricht, nach einem Gewebebildmuster sucht und einen am besten passenden Abschnitt als den lädierten Teil in den kontrastverstärkten Bildern der Arterien-Dominanzphase detektiert.

6. Medizinische Bildverarbeitungsvorrichtung (100) nach Anspruch 1, wobei das Erfassungsgerät (141, 341) weiter so konfiguriert ist, dass es ein Gewebebild des Probanden erfasst, das zusammen mit den kontrastverstärkten Bildern aufgenommen wurde, und der Detektor (144, 344) so konfiguriert ist, dass er:

den lädierten Teil im Gewebebild in der arteriell dominanten Phase detektiert, wenn der lädierte Teil in den kontrastverstärkten Bildern in der portalvenös dominanten Phase nicht detektiert wurde; und

ein Einzelbild mit der höchsten Wahrscheinlichkeit des lädierten Teils aus den Einzelbildern der kontrastverstärkten Bilder basierend auf dem Gewebebild in der arteriell dominanten Phase auswählt, in der die lädierten Teile detektiert werden,

weiter umfassend:

einen Grenzsetzer (146), der so konfiguriert ist, dass er eine Grenze zwischen dem lädierten Teil und einem nicht lädierten Teil in dem Einzelbild mit der höchsten Wahrscheinlichkeit des lädierten Teils festlegt, indem er eine Segmentierung in dem Einzelbild mit der höchsten Wahrscheinlichkeit des lädierten Teils durchführt;

einen Zeitkurvengenerator (148, 348), der so konfiguriert ist, dass er eine Bewegungskompensation in umgekehrter Zeitrichtung durchführt, ausgehend von dem Einzelbild mit der höchsten Wahrscheinlichkeit, unter Verwendung einer Region von Interesse, die den lädierten Teil und den nicht lädierten Teil beinhaltet, um eine Zeit-Intensitäts-Kurve zu erzeugen, die die zeitliche Veränderung der Kontrastmittelanreicherung im lädierten Teil anzeigt;

einen Peak-Einzelbild-Auswahlgerät (149, 349), der so konfiguriert ist, dass er ein Peak-Einzelbild, in dem die Kontrastmittelanreicherung im lädierten Teil den Höhepunkt erreicht, basierend auf der der Zeit-Intensitäts-Kurve auswählt; und

einen Befundgenerator (150, 350), der so konfiguriert ist, dass er Befunde der Kontrastmittelanreicherung im Peak-Einzelbild basierend auf der Luminanzdifferenz zwischen dem lädierten Teil und dem nicht lädierten Teil im Peak-Einzelbild erstellt.

7. Medizinische Bildverarbeitungsvorrichtung (100) nach Anspruch 6, wobei der Detektor (144, 344) so konfiguriert ist, dass er den lädierten Teil basierend auf Ausgabeergebnissen identifiziert, die durch Eingabe des Gewebebildes in ein zweites trainiertes Modell (185, 385) erhalten werden, das unter Verwendung des Gewebebildes, das den lädierten Teil beinhaltet, als Trainingsdaten erzeugt wurde.

8. Medizinische Bildverarbeitungsvorrichtung (100) nach einem der Ansprüche 1 bis 7, wobei das Erfassungsgerät (141, 341) weiter so konfiguriert ist, dass es ein kontrastverstärktes Bild des Probanden in der arteriell dominanten Phase erfasst, und der Detektor (144, 344) so konfiguriert ist, dass er einen lädierten Teil aus den kontrastverstärkten Bildern der arteriell dominanten Phase detektiert.

9. Medizinische Bildverarbeitungsvorrichtung (100) nach Anspruch 8, wobei der Detektor (144, 344) so konfiguriert ist, dass er den lädierten Teil basierend auf Ausgabeergebnissen detektiert, die durch Eingeben der kontrastverstärkten Bilder der arteriell dominanten Phase in ein drittes trainiertes Modell (186, 386) erhalten werden, das unter Verwendung der kontrastverstärkten Bilder der arteriell dominanten Phase, die den lädierten Teil beinhalten, als Trainingsdaten erzeugt wurde.

10. Medizinische Bildverarbeitungsvorrichtung (100) nach einem der Ansprüche 1 bis 9, wobei das Erfassungsgerät (141, 341) so konfiguriert ist, dass es Korrekturanweisungsinformationen für Daten erfasst, die zum Detektieren des lädierten Teils verwendet werden.

11. Medizinische Bildverarbeitungsvorrichtung (100) nach Anspruch 1, wobei das Erfassungsgerät (141, 341) so konfiguriert ist, dass es ein Gewebebild des Probanden, das vor Verabreichung des Kontrastmittels an den Probenanden aufgenommen wurde, und ein kontrastverstärktes Bild des Probanden, dem das Kontrastmittel verabreicht wurde, erfasst, und der Detektor so konfiguriert ist, dass er einen lädierten Teil, in dem das Kontrastmittel ausgewaschen wurde, im Gewebebild des Probanden, im kontrastverstärkten Bild der arteriell dominanten Phase und in kontrastverstärkten Bildern nach der portalvenös dominanten Phase detektiert.

12. Ultraschall-Diagnoseeinrichtung (1), umfassend:

die medizinische Bildverarbeitungsvorrichtung (100) nach einem der Ansprüche 1 bis 11; und

eine Ultraschallsonde (10), die so konfiguriert ist, dass sie Ultraschallwellen aussendet und ein Echo der ausgesendeten Ultraschallwellen empfängt,

wobei das Erfassungsgerät (141, 341) so konfiguriert ist, dass es die kontrastverstärkten Bilder erfasst, die basierend auf dem von der Ultraschallsonde (10) empfangenen Ultraschallecho erzeugt werden.

13. Medizinische Bildverarbeitungsvorrichtung (100) nach einem der Ansprüche 1 bis 11, wobei das Erfassungsgerät (141, 341) so konfiguriert ist, dass es die kontrastverstärkten Bilder erfasst, die von einer über ein Netzwerk angeschlossenen Modalität bereitgestellt werden.

14. Computerlesbares Speichermedium, das ein Programm speichert, das, wenn das Programm von einem Computer ausgeführt wird, den Computer veranlasst zum:

Erfassen von kontrastverstärkten Bildern eines Probanden mindestens nach Erreichen einer portalvenös dominanten Phase, wobei es sich bei den kontrastverstärkten Bildern um kontrastverstärkte Bilder des Probanden handelt, dem ein Kontrastmittel in einem Prozess von Erreichen einer postvaskulären Phase von einer arteriell dominanten Phase verabreicht wurde, wobei die kontrastverstärkten Bilder mit einer Ultraschall-Diagnoseeinrichtung (1) erzeugt werden;

Detektieren einer Stelle, an der das Kontrastmittel ausgewaschen wurde, als einen lädierten Teil in den kontrastverstärkten Bildern nach der portalvenös dominanten Phase;

Identifizieren eines Zeitpunkts, zu dem ein Detektionseinzelbild aufgenommen wurde, das ein Einzelbild der kontrastverstärkten Bilder ist, in dem der lädierte Teil unter der Vielzahl von kontrastverstärkten Bildern detektiert wurde;

**gekennzeichnet dadurch, dass** der Computer weiter veranlasst wird zum:

Festlegen einer Grenze zwischen dem lädierten Teil und einem nicht lädierten Teil im Detektionseinzelbild durch Durchführen einer Segmentierung im Detektionseinzelbild;

Bestimmen eines Auswaschungsgrads im lädierten Teil basierend auf einer Luminanzdifferenz zwischen dem lädierten Teil und dem nicht lädierten Teil im Detektionseinzelbild;

Erzeugen einer Zeit-Intensitäts-Kurve, die eine zeitliche Veränderung der Kontrastmittelanreicherung im lädierten Teil anzeigt, indem ein kontrastverstärktes Bild in einer Arterienphase in umgekehrter Zeitrichtung aus einer Portalvenenphase unter Verwendung einer Region von Interesse, die den lädierten Teil und den nicht lädierten Teil beinhaltet, bewegungskompensiert wird;

Auswählen eines Peak-Einzelbild, in dem die Kontrastmittelanreicherung im lädierten Teil den Höhepunkt erreicht, basierend auf der Zeit-Intensitäts-Kurve;

Erstellen von Befunden zur Kontrastmittelanreicherung im Peak-Einzelbild basierend auf dem Luminanzunterschied zwischen dem lädierten Teil und dem nicht lädierten Teil im Peak-Einzelbild.

**Revendications**

1. Dispositif de traitement d'images médicales (100) comprenant :

une fonction d'acquisition (141, 341) configurée pour acquérir des images à contraste amélioré d'un sujet auquel un agent de contraste a été administré au cours d'un processus d'atteinte d'une phase post-vasculaire à partir d'une phase artérielle dominante par l'intermédiaire d'une phase portale dominante, la fonction d'acquisition étant configurée pour acquérir des images à contraste amélioré au moins après que la phase portale dominante a été atteinte, les images à contraste amélioré étant générées par un appareil de diagnostic ultrasonore (1) ;

une fonction de détection (144) configurée pour détecter un site dans lequel l'agent de contraste a été éliminé comme une partie lésée dans les images à contraste amélioré après la phase portale dominante ;

une fonction d'identification de temps (145) configurée pour identifier un temps auquel une trame de détection, c'est-à-dire une trame des images à contraste amélioré dans laquelle la partie lésée est détectée parmi la pluralité d'images à contraste amélioré, a été capturée ;

**caractérisé en ce que** le dispositif de traitement d'images médicales (100) comprend en outre :

une fonction de définition de limite (146) configurée pour définir une limite entre la partie lésée et une partie non lésée dans la trame de détection en effectuant une segmentation dans la trame de détection ;

une fonction de détermination de degré de défaut (147) configurée pour déterminer un degré d'élimination dans la partie lésée sur la base d'une différence de luminance entre la partie lésée et la partie non lésée dans la trame de détection ;

une fonction de génération de courbe temporelle (148) configurée pour générer une courbe d'intensité temporelle indiquant une variation temporelle du rehaussement de l'agent de contraste dans la partie lésée au moyen d'une compensation de mouvement relative à une image à contraste amélioré pendant une phase artérielle dans le sens temporel inverse depuis une phase portale en utilisant une région d'intérêt incluant la partie lésée et la partie non lésée ;

une fonction de sélection de trame de pic (149) configurée pour sélectionner une trame de pic dans laquelle le rehaussement de l'agent de contraste atteint un pic dans la partie lésée sur la base de la courbe d'intensité temporelle ;

une fonction de création de conclusions (150, 350) configurée pour créer des conclusions du rehaussement de l'agent de contraste dans la trame de pic sur la base de la différence de luminance entre la partie lésée et la partie non lésée dans la trame de pic.

2. Dispositif de traitement d'images médicales (100) selon la revendication 1, dans lequel la fonction de détection (144, 344) est configurée pour détecter la partie lésée sur la base de résultats délivrés en sortie obtenus en entrant les images à contraste amélioré du sujet dans un premier modèle entraîné (184, 384) généré par apprentissage des images à contraste amélioré incluant la partie lésée à titre de données d'entraînement.

3. Dispositif de traitement d'images médicales (100) selon l'une quelconque des revendications 1 à 2, dans lequel la phase portale dominante est définie sur la base d'informations temporelles mesurées par une minuterie ou sur la base d'informations d'annotation définies pour un processus d'examen.

4. Dispositif de traitement d'images médicales (100) selon l'une quelconque des revendications 1 à 3, dans lequel la fonction d'acquisition (141, 341) est en outre configurée pour acquérir des informations de désignation d'une partie lésée désignée par un opérateur lorsque la fonction de détection (144, 344) détecte une pluralité de parties lésées.

5. Dispositif de traitement d'images médicales (100) selon l'une quelconque des revendications 1 à 4, dans lequel la fonction d'acquisition (141, 341) est en outre configurée pour acquérir une image d'un tissu du sujet capturée en même temps que les images à contraste amélioré, et la fonction de détection (144, 344) est configurée pour rechercher un modèle d'une image d'un tissu à une position correspondant à la partie lésée dans les images à contraste amélioré et pour détecter une portion correspondant au mieux au titre de la partie lésée dans les images à contraste amélioré de la phase artérielle dominante.

6. Dispositif de traitement d'images médicales (100) selon la revendication 1, dans lequel la fonction d'acquisition (141, 341) est en outre configurée pour acquérir une image d'un tissu du sujet capturée en même temps que les images à contraste amélioré, et la fonction de détection (144, 344) est configurée pour :

détecter la partie lésée dans l'image du tissu pendant la phase artérielle dominante lorsque la partie lésée dans les images à contraste amélioré pendant la phase portale dominante n'a pas été détectée ;

sélectionner une trame présentant une probabilité la plus élevée de contenir la partie lésée parmi les trames des images à contraste amélioré sur la base de l'image du tissu au cours de la phase artérielle dominante pendant laquelle les parties lésées sont détectées,

comprenant en outre :

une fonction de définition de limite (146) configurée pour définir une limite entre la partie lésée et une partie non lésée dans la trame présentant la probabilité la plus élevée de contenir la partie lésée en effectuant une segmentation dans la trame présentant la probabilité la plus élevée de contenir la partie lésée ;

une fonction de génération de courbe temporelle (148, 348) configurée pour effectuer une compensation de mouvement dans un sens temporel inverse en commençant par la trame présentant la probabilité la plus élevée en utilisant une région d'intérêt incluant la partie lésée et la partie non lésée pour générer une courbe d'intensité temporelle indiquant une variation temporelle du rehaussement de l'agent de contraste dans la partie lésée ;

une fonction de sélection de trame de pic (149, 349) configurée pour sélectionner une trame de pic dans laquelle le rehaussement de l'agent de contraste atteint un pic dans la partie lésée sur la base de la courbe d'intensité temporelle ; et

une fonction de création de conclusions (150, 350) configurée pour créer des conclusions du rehaussement de l'agent de contraste dans la trame de pic sur la base de la différence de luminance entre la partie lésée et la partie non lésée dans la trame de pic.

7. Dispositif de traitement d'images médicales (100) selon la revendication 6, dans lequel la fonction de détection (144, 344) est configurée pour identifier la partie lésée sur la base de résultats délivrés en sortie obtenus en entrant l'image du tissu dans un deuxième modèle entraîné (185, 385) généré en utilisant l'image du tissu incluant la partie lésée à titre de données d'entraînement.

8. Dispositif de traitement d'images médicales (100) selon l'une quelconque des revendications 1 à 7, dans lequel la fonction d'acquisition (141, 341) est en outre configurée pour acquérir une image à contraste amélioré du sujet pendant la phase artérielle dominante, et la fonction de détection (144, 344) est configurée pour détecter une partie lésée à partir des images à contraste amélioré de la phase artérielle dominante.

9. Dispositif de traitement d'images médicales (100) selon la revendication 8, dans lequel la fonction de détection (144, 344) est configurée pour détecter la partie lésée sur la base de résultats délivrés en sortie obtenus en entrant les images à contraste amélioré de la phase artérielle dominante dans un troisième modèle entraîné (186, 386) généré en utilisant les images à contraste amélioré de la phase artérielle dominante incluant la partie lésée à titre de données d'entraînement.

10. Dispositif de traitement d'images médicales (100) selon l'une quelconque des revendications 1 à 9, dans lequel la fonction d'acquisition (141, 341) est configurée pour acquérir des informations d'instruction de correction relatives à des données utilisées pour détecter la partie lésée.

11. Dispositif de traitement d'images médicales (100) selon la revendication 1, dans lequel la fonction d'acquisition (141, 341) est configurée pour acquérir une image d'un tissu du sujet capturée avant l'administration de l'agent de contraste au sujet et une image à contraste amélioré du sujet auquel l'agent de contraste a été administré, et la fonction de détection est configurée pour détecter une partie lésée dans laquelle l'agent de contraste a été éliminé dans l'image du tissu du sujet, dans l'image à contraste amélioré de la phase artérielle dominante, et dans les images à contraste amélioré après la phase portale dominante.

12. Appareil de diagnostic ultrasonore (1) comprenant :

le dispositif de traitement d'images médicales (100) selon l'une quelconque des revendications 1 à 11 ; et
une sonde ultrasonore (10) configurée pour transmettre des ondes ultrasonores et recevoir un écho des ondes ultrasonores transmises,
dans lequel la fonction d'acquisition (141, 341) est configurée pour acquérir les images à contraste amélioré générées sur la base de l'écho ultrasonore reçu par la sonde ultrasonore (10).

13. Dispositif de traitement d'images médicales (100) selon l'une quelconque des revendications 1 à 11, dans lequel la fonction d'acquisition (141, 341) est configurée pour acquérir les images à contraste amélioré fournies par une modalité connectée par l'intermédiaire d'un réseau.

14. Support de stockage lisible par ordinateur stockant un programme qui, lorsque ledit programme est exécuté par un ordinateur, amène l'ordinateur à :

acquérir des images à contraste amélioré d'un sujet au moins après qu'une phase portale dominante a été atteinte, lesdites images à contraste amélioré faisant partie d'images à contraste amélioré du sujet auquel un agent de contraste a été administré au cours d'un processus d'atteinte d'une phase post-vasculaire à partir d'une phase artérielle dominante, les images à contraste amélioré étant générées par un appareil de diagnostic ultrasonore (1) ;
détecter un site où l'agent de contraste a été éliminé comme une partie lésée dans les images à contraste amélioré après la phase portale dominante ;
identifier un temps auquel une trame de détection, c'est-à-dire une trame des images à contraste amélioré dans laquelle la partie lésée est détectée parmi la pluralité d'images à contraste amélioré, a été capturée ;
**caractérisé en ce que** l'ordinateur est en outre amené à :

définir une limite entre la partie lésée et une partie non lésée dans la trame de détection en effectuant une segmentation dans la trame de détection ;
déterminer un degré d'élimination dans la partie lésée sur la base d'une différence de luminance entre la partie lésée et la partie non lésée dans la trame de détection ;
générer une courbe d'intensité temporelle indiquant une variation temporelle du rehaussement de l'agent de contraste dans la partie lésée au moyen d'une compensation de mouvement relative à une image à contraste amélioré pendant une phase artérielle dans le sens temporel inverse depuis une phase portale en utilisant une région d'intérêt incluant la partie lésée et la partie non lésée ;
sélectionner une trame de pic dans laquelle le rehaussement de l'agent de contraste atteint un pic dans la partie lésée sur la base de la courbe d'intensité temporelle ;

créer des conclusions du rehaussement de l'agent de contraste dans la trame de pic sur la base de la différence de luminance entre la partie lésée et la partie non lésée dans la trame de pic.

FIG. 1

1

**MEDICAL IMAGE PROCESSING DEVICE** — 100

10
ULTRASONIC PROBE

COMMUNICATION INTERFACE — 110

INPUT INTERFACE — 120

DISPLAY — 130

PROCESSING CIRCUITRY — 140

ACQUISITION FUNCTION — 141

IMAGE GENERATION FUNCTION — 142

DISPLAY CONTROL FUNCTION — 143

DETECTION FUNCTION — 144

TIME IDENTIFICATION FUNCTION — 145

BOUNDARY SETTING FUNCTION — 146

DEFECT DEGREE DETERMINATION FUNCTION — 147

TIME CURVE GENERATION FUNCTION — 148

PEAK FRAME SELECTION FUNCTION — 149

FINDING CREATION FUNCTION — 150

MEMORY — 180

TISSUE IMAGE DATA — 181

CONTRAST-ENHANCED IMAGE DATA — 182

ANNOTATION INFORMATION — 183

FIRST TRAINED MODEL — 184

SECOND TRAINED MODEL — 185

THIRD TRAINED MODEL — 186

EP 4 213 093 B1

FIG. 2

FIG. 3

ARTERY
DOMINANT
PHASE

PORTAL VEIN
DOMINANT
PHASE

POST-VASCULAR
PHASE

FIG. 4

GA30　　GA35

GA31　　GA36

FIG. 5

22

FIG. 6

```
                    ┌─────────┐
                    │  START  │
                    └────┬────┘
                         │                    ◄──────────┐
                         ▼                                │
            ┌────────────────────────┐                    │
            │  ACQUIRE TISSUE IMAGE  │──── S101           │
            └───────────┬────────────┘                    │
                        │          S103                   │
                        ▼        ╱                        │
                     ╱─────────────────────╲              │
                    ╱   HAS CONTRAST MEDIUM  ╲   NO        │
                    ╲   BEEN ADMINISTERED?   ╱ ───────────┘
                     ╲─────────────────────╱
                        │ YES
                        ▼
        ┌──────────────────────────────────┐
        │ START ACQUISITION OF TISSUE IMAGE │──── S105
        │    AND CONTRAST-ENHANCED IMAGE    │
        └────────────────┬─────────────────┘
                         ▼
        ┌──────────────────────────────────┐
        │       ACQUIRE AND STORE IMAGE     │──── S107
        │      OF ARTERY DOMINANCE PHASE    │
        └────────────────┬─────────────────┘
                         ▼
        ┌──────────────────────────────────┐
        │     ACQUIRE AND STORE IMAGE OF    │──── S109
        │     PORTAL VEIN DOMINANCE PHASE   │
        └────────────────┬─────────────────┘
                         ▼
        ┌──────────────────────────────────┐
        │      ACQUIRE AND STORE IMAGE      │──── S111
        │      OF POST-VASCULAR PHASE       │
        └────────────────┬─────────────────┘
                         ▼
                    ┌─────────┐
                    │   END   │
                    └─────────┘
```

FIG. 7

FIG. 8

```
        ┌─────────┐
        │  START  │
        └────┬────┘
             │
             ▼
          S301
    ╱─────────────────╲
   ╱ HAS CONTRAST-      ╲   NO
  ╱  ENHANCED IMAGE      ╲─────┐
  ╲  BEEN ACQUIRED?      ╱     │
   ╲                    ╱      │
    ╲──────────────────╱       │
             │ YES             │
             ▼                 │
  ┌───────────────────┐        │
  │    UPDATE FIRST   │ S303   │
  │   TRAINED MODEL   │        │
  └─────────┬─────────┘        │
            │                  │
            ▼                  │
  ┌───────────────────┐        │
  │   STORE TRAINED   │ S305   │
  │  MODEL IN MEMORY  │        │
  └─────────┬─────────┘        │
            │                  │
            ▼                  │
        ┌─────────┐            │
        │   END   │            │
        └─────────┘            │
```

FIG. 9

ARTERY DOMINANT PHASE                    PORTAL VEIN DOMINANT PHASE

──────────────────────────────────────────────────────► TIME

GA41          GA46              GA51              GA56

GA40      GA45                  GA50        GA55

FIG. 10

TZ1   TZ2   TZ3

TIME

GA60   GA61   GA62   GA63   GA64

EP 4 213 093 B1

FIG. 11

**300** MEDICAL IMAGE PROCESSING DEVICE

**310** COMMUNICATION INTERFACE

**320** INPUT INTERFACE

**330** DISPLAY

PROCESSING CIRCUITRY ~**340**

ACQUISITION FUNCTION ~**341**

IMAGE GENERATION FUNCTION ~**342**

DISPLAY CONTROL FUNCTION ~**343**

DETECTION FUNCTION ~**344**

TIME IDENTIFICATION FUNCTION ~**345**

BOUNDARY SETTING FUNCTION ~**346**

DEFECT DEGREE DETERMINATION FUNCTION ~**347**

TIME CURVE GENERATION FUNCTION ~**348**

PEAK FRAME SELECTION FUNCTION ~**349**

FINDING CREATION FUNCTION ~**350**

**380**

MEMORY

TISSUE IMAGE DATA ~**381**

CONTRAST-ENHANCED IMAGE DATA ~**382**

ANNOTATION INFORMATION ~**383**

FIRST TRAINED MODEL ~**384**

SECOND TRAINED MODEL ~**385**

THIRD TRAINED MODEL ~**386**

**1** ULTRASONIC DIAGNOSTIC APPARATUS

NW

EP 4 213 093 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **LIANG X** ; **LIN L** ; **CAO Q** ; **HUANG R** ; **WANG Y.** Recognizing focal liver lesions in CEUS with dynamically trained latent structured models.. *IEEE Transactions on Medical Imaging*, 26 October 2015, vol. 35 (3), 713-27

• **KONDO S** ; **TAKAGI K** ; **NISHIDA M** ; **IWAI T** ; **KUDO Y** ; **OGAWA K** ; **KAMIYAMA T** ; **SHIBUYA H** ; **KAHATA K** ; **SHIMIZU C.** Computer-aided diagnosis of focal liver lesions using contrast-enhanced ultrasonography with perflubutane microbubbles.. *IEEE transactions on medical imaging*, 26 January 2017, vol. 36 (7), 1427-37